# EUROPEAN PATENT SPECIFICATION

(11) **EP 3 169 283 B1**
(45) Date of publication and mention of the grant of the patent: **12.09.2018**
(21) Application number: 15745391.1
(22) Date of filing: 10.07.2015
(51) Int. Cl.: A61F 5/02

(54) **PLATE FOR SAGITTAL CORRECTION OF THE SPINE**
PLATTE FÜR SAGITTALE KORREKTUR DER WIRBELSÄULE
PLAQUE POUR LA CORRECTION SAGITTALE DE LA COLONNE VERTÉBRALE

(30) Priority: 15.07.2014 DK 201470448
(43) Date of publication of application: 24.05.2017
(73) Proprietor: Hvidovre Hospital, 2650 Hvidovre (DK)
(72) Inventor: WONG, Christian, DK-2800 Kgs. Lyngby (DK); NIELSEN, Jan, DK-4654 Faxe Ladeplads (DK)
(74) Representative: Høiberg P/S
(86) International application number: PCT/DK2015/050214
(87) International publication number: WO 2016/008496

(56) References cited:
- WO-A1-96/35400
- WO-A1-2007/070946
- US-A- 5 474 523
- US-A1- 2005 209 726
- US-A1- 2013 317 400

## Description

The present invention relates to a scoliosis monopiece plate for sagittal correction of the spine, a method for manufacturing a plate for sagittal correction of the spine.

### Background of invention

Scoliosis is a medical condition in which a person's spine is curved from side to side. The definition of scoliosis is a spinal curvature to the right or left in the coronal plane.

Idiopathic adolescent scoliosis (AIS) has been described as early as 400 BC by Hippocrates. Since then, numerous studies have been conducted to clarify the aetiopathology behind AIS, suggesting a broad variety from genetic, growth, chemical, biomechanical to neuromuscular causes. So far no single causal theory for AIS has prevailed and it is still a conundrum and regarded as a multifactorial disease.

The conventional medical management of scoliosis for children and adolescents include observation, bracing and surgery. For moderate scoliosis, orthotic spinal bracing is the traditional treatment, sometimes combined with an exercise program. Bracing is normally done when the patient has bone growth remaining. In general, the coronal plane deformity is corrected by pressuring the spine 'straight' from sides of the trunk and preventing the curve from progressing to the point where surgery is recommended.

The effectiveness of these braces has been questioned by some clinicians and researchers since the published evidence to support it is inconclusive. Most of the known braces are not able to provide a lasting true correction of the spinal deformities. Most of the braces are adapted to apply relatively high counter pressure to fixate the deformities and may be uncomfortable to wear. In particular the braces with hard plastic modules, such as the Boston brace, may be relatively heavy and warm for the user to wear. These braces often demand 18 to 23 hours of daily wear through skeletal maturity.

WO 96/35400 A1 (COTRAUMA CENTRO ORTOPEDICO TRA [BR]) discloses a postural bra for protecting the breast and prevent and correct a tendency of hyper-kyphosis of the backbone. The arrangement of straps and plates are arranged to straighten the upper part of the back.
WO 2007/070946 A1 (KENDRICK RYAN JOSEPH [AU]) discloses a self-adhesive support which conforms to the human back and spans a number of joints, thereby maintaining body posture in the range of the neural spine. The support may be in the shape of an X. Correction of posture can be achieved by applying support to prevent thoracolumbar flexion.

The preamble of claim 1 is based on this document.

### Summary of invention

The present invention relates to a scoliosis monopiece plate to be placed on the back of the user. The plate treats the cause of the scoliosis rather than the medical condition that is the consequence. The theoretical basis for the functioning of the plate is that AIS is developed as a consequence of the spine growing into a mechanically unstable column and seems to progress in a self-sustaining 'vicious cycle' due to the effect of gravity and asymmetric loads in a growth-modulating buckling-like manner. In this regard adolescent females distinguish themselves generally from males by a significantly increased and earlier thoracic growth. These factors coincide with a temporal sagittal flattening of thoracic vertebrae for adolescent females. The sagittal flattening contributes to the overall rotational instability of the spine. Therefore, in order to counteract this rotational instability, the present invention reduces the sagittal flattening of thoracic vertebrae. The present invention relates to a monopiece plate that is shaped to fit tightly on the back of a user. The upper section of the plate is rounded in the sagittal plane, wherein said upper section is bent more than the corresponding upper back of the user without the plate being attached, thereby configuring the plate to force the thoracic spine of the user to bend forward in the sagittal plane, thereby creating an external hyperkyphosing thoracic posture.

As mentioned one advantage of the disclosed plate is that it treats the cause of the scoliosis rather than the medical condition that is the consequence. Another advantage is that the plate can be made significantly smaller and lighter than for example a traditional Boston brace, thus more comfortable to wear. The plate is placed directly on the back of the user and is preferably supported by a supplementary soft brace or clothes. The soft brace may have different shapes and materials but is preferable designed to give solid support for the monopiece plate, and at the same time have a light, discrete appearance. The soft brace may be made of for example a thin, elastic material, wrapped around the torso and/or shoulders. It should be remembered that the plate (or a traditional brace) is to be worn during a significant portion of the day.
The present invention also relates to a method for manufacturing a scoliosis monopiece plate, to fit tightly on the back of a user, comprising a lower section and an upper section, wherein the upper section is rounded in the sagittal plane. The inventors have realized that the process of manufacturing an individually fitted monopiece plate can be automatized to some extent. The method comprises the steps of creating a three-dimensional image of the back of the user (for example by scanning and/or photographing and/or drawing and/or casting the back of the user), creating a three-dimensional image of a plate based on the three-dimensional image of the back, and finally forming a scoliosis monopiece plate according to the three-dimensional image of the plate (for example by carving, cutting, grinding or molding). The upper section of the plate should, additionally, be bent in the sagittal plane. This can be achieved either by (virtually) bending the upper section of the plate of the image or by bending the upper section of the (physical) monopiece plate.

### Description of Drawings

The invention will in the following be described in greater detail with reference to the accompanying drawings. The drawings are exemplary and are intended to illustrate some of the features of the present method and plate and are not to be construed as limiting to the presently disclosed invention.
**Fig. 1** shows a front view of a monopiece plate, configured to force the thoracic spine of the user to bend forward in the sagittal plane, wherein the upper section is divided in the longitudinal direction into a number of sub-sections.
**Fig. 2** shows a front view of a monopiece plate, configured to force the thoracic spine of the user to bend forward in the sagittal plane, wherein the upper section is divided in the longitudinal direction into a number of sub-sections, with spacing between the sub-sections.
**Fig. 3** shows a side view of a monopiece plate next to a user, said plate configured to force the thoracic spine of the user to bend forward in the sagittal plane, wherein the upper section of the plate is rounded in the sagittal plane.

### Definitions

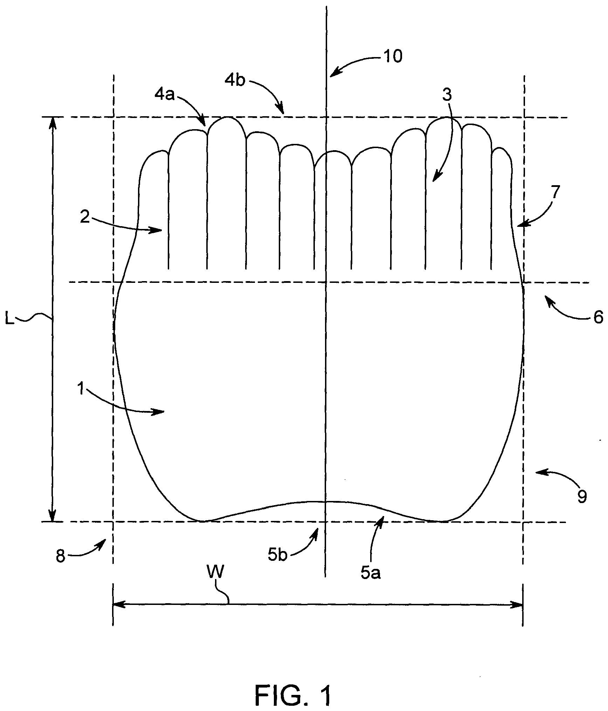

**Upper section** is defined as the upper part of the plate when the plate is attached on the back of the user. Hence, it is the upper section of the plate that is rounded in the sagittal plane to provide the force that bends the thoracic spine of the user forward in the sagittal plane. Fig. 1 shows an example of a monopiece plate wherein the boundary 6 divides the plate in an upper and a lower section.

**Lower section** is defined as the lower part of the plate when the plate is attached on the back of the user. Fig. 1 shows an example of a monopiece plate wherein the boundary 6 divides the plate in an upper and a lower section.

**Upper edge** is defined as the upper edge of the upper section of the plate when the plate is attached on the back of the user. Hence, the upper edge is the edge closest to the head of the user when the plate is attached on the back of the user.

**Lower edge** is defined as the lower edge of the lower section of the plate when the plate is attached on the back of the user.

**Upper horizontal extension of the upper edge** is defined as a line horizontally extended from the upmost point on the upper edge of the plate. Horizontal is defined as transversal to a vertical central axis of the plate.

**Lower horizontal extension of the lower edge** is defined as a line horizontally extended from the lowest point on the lower edge of the plate.

**Length** is defined as the distance between the upper horizontal extension of the upper edge and the lower horizontal extension of the lower edge of the plate. In fig. 1 the length of the plate is marked 'L'.

**Width** is defined as the distance between the side edge extensions of the plates. In fig. 1 the width of the plate is marked 'W' and is the distance between the side edge extensions 8 and 9.

**Sagittal plane** is defined as a vertical plane which passes from anterior to posterior of the body, dividing the body into right and left halves.

**Coronal plane** (also known as the frontal plane) is any vertical plane that divides the body into ventral and dorsal (belly and back) sections.

### Detailed description of the invention

The presently disclosed invention relates to a scoliosis monopiece plate, to fit tightly on the back of a user, comprising a lower section and an upper section, wherein the upper section is rounded in the sagittal plane, wherein said upper section is bent more than the corresponding upper back of the user without the plate being attached, thereby configuring the plate to force the thoracic spine of the user to bend forward in the sagittal plane. Conventional brace treatment has been aimed at treating the apparent coronal plane deformity, wherein the coronal plane deformity is corrected by pressuring the spine 'straight' from sides of the trunk. Examples of conventional braces include for example the Boston brace and the Providence brace. The development of scoliosis is typically associated with adolescent females. Principally, AIS has developed as a consequence of the spine growing into a mechanically unstable column and seems to progress in a self-sustaining 'vicious cycle' due to the effect of gravity and asymmetric loads in a growth-modulating buckling-like manner. The spinal growth spurt for both sexes coincides with adolescence, but adolescent females distinguish themselves from males by a significantly increased and earlier thoracic growth. These factors coincide with a temporal sagittal flattening of thoracic vertebrae for adolescent females. This contributes to the overall rotational instability of the spine. One aspect of the presently disclosed invention is therefore to treat the primary cause of the condition rather than the consequence. By forcing thoracic spine of the user to bend forward in the sagittal plane, a kyphosis effect of thoracic spine is achieved, which counteracts the rotational instability. The presently disclosed monopiece plate is shaped to fit tightly on the back of a user; however, the rounded upper section of the plate is further bent slightly forward to create a force on thoracic vertebrae, thereby creating an external hyperkyphosing thoracic posture. The plate according to the present invention is shaped to fit tightly on the back of a user, more precisely to an external area corresponding to the position of thoracic vertebrae, which, as stated above, is also the target to bend. However, the plate may also extend beyond the upper back in all directions in order to achieve optimal support. The plate is typically placed on the upper back of the user to create a pressure on the upper part of thoracic vertebrae. However, a portion of the plate may be configured to be placed on the lower back of the user. The reason for doing this is typically to target the sagittal flattening of the lower part of the thoracic spine. This means that the purpose is still the same, i.e. to bend the thoracic spine forward in the sagittal plane, but the bending is performed where it makes most sense based on the shape of the spine of the user. The main advantage of the disclosed plate is that it treats a cause of the scoliosis rather than the medical condition that is the consequence. Another advantage is that the plate can be made significantly smaller and lighter than for example a traditional Boston brace, thus more comfortable to wear. In a preferred embodiment of the present invention, the plate is placed directly on the back of the user, typically supported by a supplementary soft brace or clothes, which is further explained below. The plate may furthermore be adapted specifically to one user, or, in another embodiment, produced to fit a group of users. The latter can be achieved by having a number of mass-producible sizes and shapes of the plate. Shape in this context has a broad meaning, including the general shape of the plate that makes the plate fit on the back and feel comfortable for the user, the roundness of the rounded upper section and the angle in which the upper section is bent forward.

### Support element(s)

In order to keep the plate at a fixed position on the back of the user, supplementary support is typically needed, preferably clothes or a supplementary soft brace that holds the plate in a fixed position. Such a soft brace may be designed to have a discrete appearance, for example a thin, soft material wrapped around the torso, neck, arm(s) or shoulder(s). It may cover the presently disclosed plate, not only to give support but also to hide it. The supporting soft brace may also comprise strap(s), belt(s) or band(s) to tighten and fixate the plate. The bands may be elastic and could be made of for example silicon. The support could also be designed as clothing, thereby reducing the need for using both external support and clothes. In one embodiment the soft brace comes as a part of the presently disclosed plate, i.e. the plate further comprises a soft brace configured to hold the plate. Both the supporting soft brace and clothing may be made of an elastic material, such as spandex. The advantage of using such a material for supporting the plate is that it is well suited for holding the plate in a fixed position and still comfortable to wear. In one embodiment the soft brace is configured to provide proprioceptive pressure feedback to the user. Proprioception is the sense of the relative position of neighbouring parts of the body and strengths of effort being employed in the movement. In this context the proprioceptive pressure from the soft brace can help the user to vary muscle contraction and thereby stabilize the spine. In one embodiment the supplementary soft brace is configured to de-rotate thoracic vertebrae around the axis of said thoracic vertebrae. The soft brace is configured to pull the upper back of the user to the left or to the right to achieve a de-rotation of thoracic vertebrae in order to counteract the spinal rotation that is associated with scoliosis. By pulling the upper back to the left or right, thereby counteracting the spinal rotation, the soft brace can assist in holding the spine in a more neutral position

In one embodiment of the presently disclosed plate the clothing, strap, band or belt is configured to further adjust the bending of the thoracic spine in the sagittal plane. As stated, these elements can be made of e.g. an elastic material. They do not necessarily have the function of holding the plate in a fixed position - they could also be employed to assist the plate in forcing the thoracic spine of the user to bend forward in the sagittal plane. These extra elements can be considered to be elements for adjusting the force on the thoracic spine. In one embodiment the clothing, strap, band or belt is/are adjusted based on information from visual indicator(s) on the plate and/or stretch sensor(s) and/or bend sensor(s). This can be done either directly by an integrated mechanism, or indirectly by a means for processing the measured data and controlling and adjusting a mechanism for changing either the place directly or the assisting clothing/strap/band. In one embodiment there is unit connected to indicators and/or stretch and/or bend sensors. The user can then either evaluate the measured data or adjust the plate and/or clothing/strap/band manually or by means of a control unit.

A further aspect of the presently disclosed invention relates to the plate being configured to be supported externally by a hard brace. Such a hard brace may be wrapped around the body of the user, and it may be configured to apply pressure to the spine from sides of the trunk. This embodiment is particularly advantageous for users that would like to combine the main advantage of the present invention (treating the cause of the scoliosis by bending the thoracic spine in the sagittal plane and thereby reduce the rotational instability) with the conventional way of dealing with the lateral deformity by pressuring the spine 'straight' from sides of the trunk.

### Shape

As mentioned, the rounded section of the plate, which is the upper section of the plate, should be further bent forward in the sagittal plane in order to achieve a kyphosis effect of thoracic spine. 'Further bent forward' in this context shall be interpreted as the rounded section being bent more than the upper back without the plate attached. This is what creates the force on thoracic vertebrae. The area 3 on the upper back of the user in fig.1 is an example of where the force can be applied to achieve a kyphosis effect of thoracic spine. In this regard the lower section of the plate can be seen as a fixed section, and in relation to this fixed section the upper rounded section should be bent between 3° and 15° forward in the sagittal plane, for example 3°, 4°, 5°, 6°, 7°, 8°, 9°, 10°, 11 °, 12°, 13°, 14° or 15°. The bending angle should be chosen such that a certain pressure is created on thoracic vertebrae.

Thoracic vertebrae compose the middle segment of the vertebral column, between the cervical vertebrae and the lumbar vertebrae. In humans, there are twelve thoracic vertebrae, T1-T12. The kyphotic angle is defined as the kobb angle between T1 and T12, 25-40°typically being considered normal. Because of temporary sagittal flattening of thoracic vertebrae, adolescent females may have a curve that measures less than the normal values. In the presently disclosed invention, the rounded section of the plate may be shaped such that the curvature corresponds to a predefined kyphotic angle of thoracic vertebrae between 5° and 25°, for example 5°, 6°, 7°, 8°, 9°, 10°, 11°, 12°, 13°, 14°, 15°, 16°, 17°, 18°, 19°, 20°, 21°, 22°, 23°, 24° or 25°. By shaping the rounded section of the plate after a preferred kyphotic angle rather than exactly after the actual kyphotic angle of the user, the external pressure that is to create a kyphosis effect of thoracic spine can be achieved.

In one embodiment of the presently disclosed invention the upper section constitutes between 25% and 75% of the total area of the plate, such as between 30% and 70%, or such as between 35% and 65%, or such as between 40% and 60% of the total area of the plate, for example 25%, 26%, 27%, 28%, 29%, 30%, 31%, 32%, 33%, 34%, 35%, 40%, 45%, 50%, 55%, 55%, 60%, 65%, 70%, or 75%.

As mentioned, one aspect of the presently disclosed invention relates to the plate being light and comfortable to wear. In order for the plate to fit on the back of the user, the length of the plate should be in the range of 200 mm - 500 mm, such as in the range of 250 mm - 450 mm, or such as in the range of 250 mm - 400 mm, for example 200 mm, 205 mm, 210 mm, 215 mm, 220 mm, 230 mm, 240 mm, 250 mm, 260 mm, 270 mm, 280 mm, 290 mm, 300 mm, 350 mm, 400 mm, 450 mm, or 500 mm.

The lower section is preferably shaped to fit tightly on the lower part of the upper back of the user, but may also extend to the lower back of the user. The lower section is typically substantially straight, but may also be slightly curved to fit the upper or lower back.

Another aspect of the presently disclosed invention relates to the rounded section of the place being elastic. If the section that creates the pressure on thoracic vertebrae is elastic, the pressure is maintained even if the user moves the back such that it temporarily does not have the normal shape. To some extent it also means that the plate does not have to be manufactured as precisely as a plate without elasticity. This means that the plate can be mass-produced in a number of predefined sizes and shapes, which may be used by patients without a need to have the exact measurement of the individual.

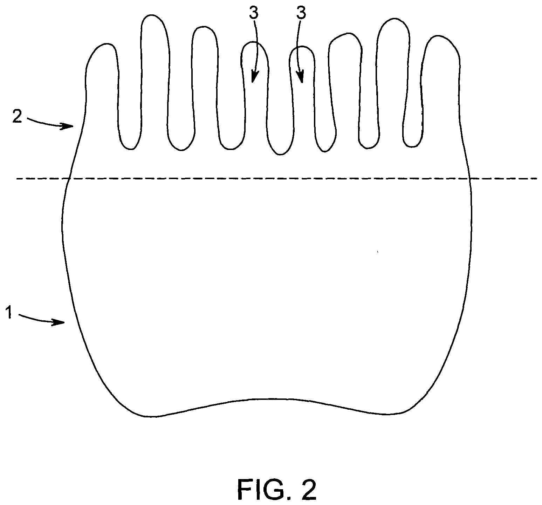

In another embodiment, the rounded section is divided in the longitudinal direction into a predefined number of elastic sub-sections. This is another advantage related to flexibility of the section of the plate that provides the pressure on thoracic vertebrae. By splitting the rounded section into a number of sub-sections, wherein each sub-section can contribute independently, a more flexible plate is obtained in terms of distributing the total force evenly. There may be spacing between the sub-sections, as illustrated in fig. 2.

In one embodiment of the present invention the lower edge of the plate is configured to be placed such that it rests on the pelvic bone. One reason for doing this is that the force on thoracic vertebrae is greater with a longer lever, and the pelvic bone constitutes a suitable lower limit for the plate. This part of the plate could potentially also help reducing the lumbar lateral instability by providing support to the lumbar spine. Alternatively, the lower edge of the plate is configured to be placed above the pelvic bone, resulting in a shorter lever but a lighter plate. The upper edge of the plate is typically configured to be placed below the cervical vertebrae.

In another embodiment of the present invention, the plate further comprises a rigid support element extending from the lower edge of the plate, wherein the support is configured to rest on the pelvic bones. This can be seen as an alternative to having a plate that rests directly on the pelvic bone. The support element can be made of a material suitable for giving support and can be designed to give maximum support from the pelvic bone.

### Materials

A shape-memory alloy (SMA, smart metal, memory metal, memory alloy, muscle wire, smart alloy) is an alloy that 'remembers' its original shape and that when deformed returns to its pre-deformed shape when heated. In one embodiment of the invention the plate is made of shape-memory alloy, such as Cu-Al-Ni, Ni-Ti, Fe-Mn-Si or Cu-Zn-Al. Such a material in the present invention is beneficial since it means that the plate can be adjusted for a tight fit on the back of the user every time it is attached. After use the plate may return to its original shape.

It is important that the plate is light since it may be worn for longer periods. Therefore the plate is preferably made of a light material, selected from a group of plastics, such as polyethylene, polypropylene, polycarbonate (PC) or polymethylmethacrylate (PMMA). However, other may also be used. Preferably, the plate is made as thin in order to provide a discrete appearance. Furthermore, the plate can be painted in an individual colour, chosen by the user.

In one embodiment of the present invention the plate further comprises a soft coating, such as yarn, polyurethane-coated textile, temperature responsive textile, or any textile. The soft coating has the purpose of making the plate more comfortable to wear.

### Referencing

In one embodiment of the present invention the plate further comprises at least one reference element for indicating a bending angle between the upper section and the lower section. It is important that the plate has the correct angle when the user wears the plate. Since there is a counter-force from the body of the user when the plate is worn, it may be useful to know the actual bending angle and other angles when the force of the plate and the counter-force of the user's body are applied. In one embodiment the reference elements are visual indicators, such as drawn lines, marks or similar on the plate. Alternatively, there may be stretch and/or bend sensors attached to the plate to indicate the bending angle, either as a an angle, or as an angle in relation to a starting point.

### Electric stimulation

In a further embodiment the plate further comprises an integrated electric stimulation element. Alternatively, the electric stimulation element can be a separate element. Electrical stimulation should be construed to include a range of purposed such as transcutaneous electrical nerve stimulation (TENS) or electrical muscle stimulation (EMS). In a preferred embodiment the electric stimulation is transferred to skin by means of pads, such as gel pads, configured to be attached to the skin. In one embodiment the electric stimulation element(s) is/are connected to a stimulation control unit for controlling the at least one electric stimulation element.

### Logging and activity monitoring

Since correcting scoliosis is typically a long process, the present disclosure further relates to the logging and monitoring of a number of parameters, some of which can be considered to be relatively long term. It may be required, or at least beneficial, to wear the plate a substantial part of the day. In one embodiment the plate further comprises a logging element and/or an activity monitoring element. The logging data can for example collecting information regarding the daily use, which can be used by medical staff to evaluate the progress of the scoliosis. The data can also be used for statistical analysis, for example by evaluating on a number of patients how different wearing patterns or configurations of the plate correlates with the progress of the scoliosis. Preferably, the scoliosis itself is also be measured and monitored.

### Manufacturing

The present invention also relates to a method for manufacturing a scoliosis monopiece plate, to fit tightly on the back of a user, comprising a lower section and an upper section, wherein the upper section is rounded in the sagittal plane, such that the plate is configured to force the thoracic spine of the user to bend forward in the sagittal plane. The method comprises the steps of:
- creating a three-dimensional image of the back of at least one user;
- creating a three-dimensional image of a plate based on the three-dimensional image of the back;
- forming a scoliosis monopiece plate according to the three-dimensional image of the plate.

The inventors have realised that the process of manufacturing a monopiece place can be automatized to some extent. In order to manufacture an individually fitted plate it is necessary to have the physical measures of the back of the user. The first step of the method is to create a three-dimensional image of the back of the user. For this purpose a 3D-scanner can be used. Another option is to provide a number of photos from different angles, which can be translated by a machine to a three-dimensional image of the back of the user. The back may also be photographed with x-rays. Based on the three-dimensional image of the back it is possible to create an image (i.e. a virtual model) of the plate to be manufactured. Based on the image of the plate it is then possible to manufacture the plate, for example by carving, cutting, grinding or molding. This process may be automatic or manual. As mentioned the plate should be configured to force the thoracic spine of the user to bend forward in the sagittal plane. This can be achieved by (virtually) bending the upper section of the plate of the image between 3° and 15°, for example 3°, 4°, 5°, 6°, 7°, 8°, 9°, 10°, 11°, 12°, 13°, 14° or 15°, forward in the sagittal plane in relation to a fixed lower section of the plate of the image, or by (virtually) bending the three-dimensional image of the plate such that the curvature of the upper section of the plate of the image corresponds to a predefined kyphotic angle of thoracic vertebrae between 5° and 25°. Alternatively, it can be achieved by bending the upper section of the monopiece plate between 3° and 15°, for example 3°, 4°, 5°, 6°, 7°, 8°, 9°, 10°, 11°, 12°, 13°, 14° or 15°, forward in the sagittal plane in relation to a fixed lower section of the monopiece plate, or by bending the upper section of the monopiece plate, such that the curvature corresponds to a predefined kyphotic angle of thoracic vertebrae between 5° and 25°. Manufacturing of the monopiece plate such that it fits a group of users is also possible. This enables that the plate can be mass-produced in a number of predefined sizes and shapes based on the image of the back of at least one user as described above.

### Examples

**Fig. 1** shows a front view of an embodiment of a monopiece plate, configured to force the thoracic spine of the user to bend forward in the sagittal plane. The plate is divided into a lower section 1 and an upper section 2 by a boundary 6. The boundary 6 does not have a technical function other than defining the boundary between the upper and lower section of the plate. 4a is the upper edge of the plate and 4b is the upper horizontal extension of the upmost point on the upper edge 4a (drawn as a horizontal dotted line). Upper in this context should be interpreted as in relation to a line transversal to the central axis 10. In this case it can be seen that the contour of the upper edge 4a is not the same as the upper horizontal extension 4b (horizontal dotted line). Similarly there is a lower edge 5a and a lower horizontal extension 4b. The upper section is divided in the longitudinal direction into a number of sub-sections 3. 7 in this example illustrates a shape of the plate that allows the arm of the user to move. Side edge extension 8 and 9 are extensions of the leftmost and rightmost points of the side edges of the plate. The extensions are parallel to the central axis 10.

**Fig. 2** shows a front view of a monopiece plate similar to the plate in fig.1, configured to force the thoracic spine of the user to bend forward in the sagittal plane, wherein the upper section is divided in the longitudinal direction into a number of sub-sections, with spacing between the sub-sections, comprising a lower section 1 and an upper section 2. In this example there is spacing between the sub-sections 3.

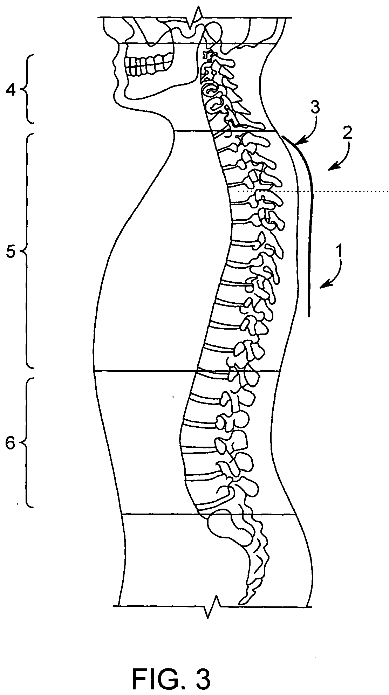

**Fig. 3** shows a side view of a monopiece plate next to a user, comprising a lower section 1 and an upper section 2. The rounded upper part forces the thoracic spine of the user to bend forward in the sagittal plane by providing a force at the area 3 of the upper back. 4, 5, and 6 indicate the cervical vertebrae, thoracic vertebrae and lumbar vertebrae respectively. The plate is placed at the upper part of thoracic vertebrae and is configured to force the thoracic spine of the user to bend forward in the sagittal plane.

## Claims

1. A scoliosis monopiece plate, to fit tightly on the back of a user, comprising a lower section (1) and an upper section (2), wherein the upper section (2) is rounded in the sagittal plane, **characterized in that** said upper section (2) is bent more than the corresponding upper back of the user without the plate being attached, thereby configuring the plate to force the thoracic spine of the user to bend forward in the sagittal plane.

2. The plate according to any of the preceding claims, wherein the upper section (2) is divided in the longitudinal direction into at least two sub-sections (3).

3. The plate according to any of the preceding claims, further comprising at least one reference element for indicating a bending angle between the upper section and the lower section.

4. The plate according to claim 3, wherein the reference element is/are visual indicator(s) on the plate and/or stretch sensor(s) and/or bend sensor(s).

5. The plate according to any of the preceding claims, wherein the upper section (2) is bent between 3° and 15° forward in the sagittal plane in relation to a fixed lower section (1) of the plate, or wherein the upper section (2) is shaped such that the curvature corresponds to a predefined kyphotic angle of thoracic vertebrae between 5° and 25°.

6. The plate according to any of the preceding claims, further comprising clothing, and/or at least one soft brace and/or at least one strap, and/or at least one band, and/or at least one belt, configured to hold the plate.

7. The plate according to claim 6, wherein the clothing, strap, band or belt is configured to further adjust the bending of the thoracic spine in the sagittal plane.

8. The plate according to any of claims 6-7, wherein the clothing, strap, band or belt is/are adjusted based on information from the visual indicator(s) on the plate and/or stretch sensor(s) and/or bend sensor(s).

9. The plate according to any of claims 6-8, further comprising a bending control unit for controlling and adjusting the clothing, strap, band or belt.

10. The plate according to any of the preceding claims, further comprising at least one electric stimulation element integrated in the plate, preferably wherein the at least one electric stimulation element is/are pads, preferably gel pads, configured to be attached to the skin.

11. The plate according to claim 10, further comprising a stimulation control unit for controlling the at least one electric stimulation element.

12. The plate according to any of the preceding claims, further comprising a logging element and/or an activity monitoring element, preferably wherein the logging element and/or activity monitoring element is/are configured to log and/or the wearing times by the user and/or status regarding the scoliosis.

13. A method for manufacturing a scoliosis monopiece plate according to any of claims 1-12, to fit tightly on the back of a user, comprising a lower section (1) and an upper section (2), wherein the upper section (2) is rounded in the sagittal plane, said upper section (2) being bent more than the corresponding upper back of the user without the plate being attached, thereby configuring the plate to force the thoracic spine of the user to bend forward in the sagittal plane, comprising the steps of:
- creating a three-dimensional image of the back of at least one user;
- creating a three-dimensional image of a plate based on the three-dimensional image of the back;
- forming the scoliosis monopiece plate according to the three-dimensional image of the plate.

14. The method according to claim 13, further comprising the step: bending the upper section of the monopiece plate between 3° and 15° forward in the sagittal plane in relation to a fixed lower section of the monopiece plate.

15. The method according to claims 13-14, wherein the three-dimensional image of the plate is modified such that the curvature of the upper section of the plate of the image corresponds to a predefined kyphotic angle of thoracic vertebrae between 5° and 25°.

## Patentansprüche

1. Einstückige Skolioseplatte, um fest auf den Rücken eines Benutzers zu passen, die einen unteren Teil (1) und einen oberen Teil (2) umfasst, wobei der obere Teil (2) in der Sagittalebene abgerundet ist, **dadurch gekennzeichnet, dass** der obere Teil (2) gebogener ist als der entsprechende obere Rücken des Benutzers, ohne dass die Platte angebracht ist, wodurch die Platte dazu konfiguriert ist, die Brustwirbelsäue des Benutzers zum Biegen nach vorne in der Sagittalebene zu zwingen.

2. Platte nach einem der vorhergehenden Ansprüche, wobei der obere Teil (2) in der Längsrichtung in mindestens zwei Unterteile (3) geteilt ist.

3. Platte nach einem der vorhergehenden Ansprüche, die ferner mindestens ein Referenzelement umfasst, um einen Biegewinkel zwischen dem oberen Teil und den unteren Teil anzugeben.

4. Platte nach Anspruch 3, wobei das Referenzelement visuelle(r) Indikator(en) auf der Platte und/oder Dehnungssensor(en) und/oder Biegesensor(en) ist/sind.

5. Platte nach einem der vorhergehenden Ansprüche, wobei der obere Teil (2) zwischen 3 ° und 15 ° nach vorne in der Sagittalebene in Bezug zu einem festen unteren Teil (1) der Platte gebogen ist, oder wobei der obere Teil (2) derart geformt ist, dass die Krümmung einem vorbestimmten kyphotischen Winkel von Brustwirbeln von zwischen 5 ° und 25 ° entspricht.

6. Platte nach einem der vorhergehenden Ansprüche, die ferner Kleidung und/oder mindestens eine weiche Stütze und/oder mindestens einen Gurt und/oder mindestens ein Band und/oder mindestens einen Riemen umfasst, die dazu konfiguriert sind, die Platte zu halten.

7. Platte nach Anspruch 6, wobei die Kleidung, der Gurt, das Band oder der Riemen dazu konfiguriert ist, ferner das Biegen der Brustwirbelsäure in der Sagittalebene einzustellen.

8. Platte nach einem der Ansprüche 6-7, wobei die Kleidung, der Gurt, das Band oder der Riemen auf Grundlage von Informationen von dem/den visuellen Indikator(en) auf der Platte und/oder Dehnungssensor(en) und/oder Biegesensor(en) eingestellt ist/sind.

9. Platte nach einem der Ansprüche 6-8, die ferner eine Biegesteuerungseinheit zum Steuern und Einstellen der Kleidung, des Gurts, des Bands oder des Riemens umfasst.

10. Platte nach einem der vorhergehenden Ansprüche, die ferner mindestens ein elektrisches Stimulierungselement umfasst, das in die Platte integriert ist, vorzugsweise, wobei mindestens ein elektrisches Stimulierungselement Kontaktflächen ist/sind, vorzugsweise Gelkontaktflächen, die dazu konfiguriert sind, an der Haut angebracht zu sein.

11. Platte nach Anspruch 10, die ferner eine Stimulationssteuerungseinheit umfasst, um das mindestens eine elektrische Stimulationselement zu steuern.

12. Platte nach einem der vorhergehenden Ansprüche, die ferner ein Aufzeichnungselement und/oder ein Aktivitätsaufzeichnungselement umfasst, vorzugsweise, wobei das Aufzeichnungselement und/oder Aktivitätsaufzeichnungselement dazu konfiguriert ist/sind, und/oder die Tragezeiten durch den Benutzer und/oder den Zustand in Bezug auf die Skoliose aufzuzeichnen.

13. Verfahren zum Herstellen einer einstückigen Skolioseplatte nach einem der Ansprüche 1-12, um fest auf den Rücken eines Benutzers zu passen, die einen unteren Teil (1) und einen oberen Teil (2) umfasst, wobei der obere Teil (2) in der Sagittalebene abgerundet ist, **dadurch gekennzeichnet, dass** der obere Teil (2) gebogener ist als der entsprechende obere Rücken des Benutzers, ohne dass die Platte angebracht ist, wodurch die Platte dazu konfiguriert ist, die Brustwirbelsäue des Benutzers zum Biegen nach vorne in der Sagittalebene zu zwingen, das die folgenden Schritte umfasst:
- Erzeugen eines dreidimensionalen Bilds des Rückens von mindesten einem Benutzer;
- Erzeugen eines dreidimensionalen Bilds einer Platte auf Grundlage des dreidimensionalen Bilds des Rückens;
- Bilden der einstückigen Skolioseplatte gemäß dem dreidimensionalen Bild der Platte.

14. Verfahren nach Anspruch 13, das ferner den folgenden Schritt umfasst: Biegen des oberen Teils der einstückigen Platte zwischen 3 ° und 15 ° nach vorne in der Sagittalebene in Bezug auf einen festen unteren Teil der einstückigen Platte.

15. Verfahren nach Anspruch 13-14, wobei das dreidimensionale Bild der Platte derart modifiziert ist, dass die Krümmung des oberen Teils der Platte des Bilds einem vorbestimmten kyphotischen Winkel der Brustwirbel von zwischen 5 ° und 25 ° entspricht.

## Revendications

1. Plaque monobloc pour scoliose, qui s'ajuste étroitement au dos d'un utilisateur, comprenant une section inférieure (1) et une section supérieure (2), dans laquelle la section supérieure (2) est arrondie dans le plan sagittal, **caractérisée en ce que** ladite section supérieure (2) est pliée davantage que le dos supérieur correspondant de l'utilisateur sans que la plaque ne soit fixée, de telle sorte que la plaque est configurée pour forcer la colonne thoracique de l'utilisateur à se plier vers l'avant dans le plan sagittal.

2. Plaque selon l'une quelconque des revendications précédentes, dans laquelle la section supérieure (2) est divisée dans la direction longitudinale dans au moins deux sous-sections (3).

3. Plaque selon l'une quelconque des revendications précédentes, comprenant en outre au moins un élément de référence pour indiquer un angle de flexion entre la section supérieure et la section inférieure.

4. Plaque selon la revendication 3, dans laquelle l'élément de référence est/sont un (des) indicateur(s) visuel(s) sur la plaque et/ou un (des) capteur(s) d'allongement et/ou un (des) capteur(s) de flexion.

5. Plaque selon l'une quelconque des revendications précédentes, dans laquelle la section supérieure (2) est pliée entre 3° et 15° vers l'avant dans le plan sagittal relativement à une section inférieure fixée (1) de la plaque, ou dans laquelle la section supérieure (2) est façonnée de manière à ce que la courbure corresponde à un angle cyphotique prédéfini des vertèbres thoraciques entre 5° et 25°.

6. Plaque selon l'une quelconque des revendications précédentes, comprenant en outre une garniture, et/ou au moins un corset souple et/ou au moins une sangle, et/ou au moins une bande, et/ou au moins une ceinture, configuré pour maintenir la plaque.

7. Plaque selon la revendication 6, dans laquelle la garniture, la sangle, la bande ou la ceinture est configurée pour en outre ajuster la flexion de la colonne thoracique dans le plan sagittal.

8. Plaque selon l'une quelconque des revendications 6 et 7, dans laquelle la garniture, la sangle, la bande ou la ceinture est/sont ajustées sur la base d'informations de l'indicateur (des indicateurs) visuel (s) sur la plaque et ou du (des) capteur(s) d'allongement et/ou du (des) capteur(s) de flexion.

9. Plaque selon l'une quelconque des revendications 6 à 8, comprenant en outre une unité de commande de flexion pour commander et ajuster la garniture, la sangle, la bande ou la ceinture.

10. Plaque selon l'une quelconque des revendications précédentes, comprenant en outre au moins un élément de stimulation électrique intégré dans la plaque, de préférence dans laquelle l'au moins un élément de stimulation électrique est/sont des tampons, de préférence des tampons de gel, configurés pour être fixés à la peau.

11. Plaque selon la revendication 10, comprenant en outre une unité de commande de stimulation pour commander l'au moins un élément de stimulation électrique.

12. Plaque selon l'une quelconque des revendications précédentes, comprenant en outre un élément de connexion et/ou un élément de surveillance de l'activité, de préférence dans laquelle l'élément de connexion et/ou l'élément de surveillance de l'activité est/sont configurés pour se connecter et/ou le temps d'utilisation par l'utilisateur et/ou le statut relativement à la scoliose.

13. Procédé de fabrication d'une plaque monobloc pour scoliose selon l'une quelconque des revendications 1 à 12, qui s'ajuste étroitement au dos d'un utilisateur, comprenant une section inférieure (1) et une section supérieure (2), dans laquelle la section supérieure est arrondie (2) dans le plan sagittal, ladite section supérieure (2) étant pliée davantage que le dos supérieur correspondant de l'utilisateur sans que la plaque ne soit fixée, de telle sorte que la plaque est configurée pour forcer la colonne thoracique de l'utilisateur à se plier vers l'avant dans le plan sagittal, comprenant les étapes de :
- création d'une image en trois dimensions du dos d'au moins un utilisateur ;
- création d'une image en trois dimensions d'une plaque sur la base de l'image en trois dimensions du dos ;
- formation d'une plaque monobloc pour scoliose selon l'image en trois dimensions de la plaque.

14. Procédé selon la revendication 13, comprenant en outre l'étape : de flexion de la section supérieure de la plaque monobloc entre 3° et 15° vers l'avant dans le plan sagittal relativement à une section inférieure fixée de la plaque monobloc.

15. Procédé selon les revendications 13 et 14, dans lequel l'image en trois dimensions de la plaque est modifiée de telle sorte que la courbure de la section supérieure de la plaque de l'image correspond à un angle cyphotique prédéfini des vertèbres thoraciques entre 5° et 25°.
